# EUROPEAN PATENT SPECIFICATION

(11) **EP 2 367 782 B1**
(45) Date of publication and mention of the grant of the patent: **13.02.2013**
(21) Application number: 09801670.2
(22) Date of filing: 22.12.2009
(51) Int. Cl.: C07C 209/88, C07C 59/50, C07D 223/16, C07C 213/10, C07C 217/58, C07C 217/74

(54) **PROCESS FOR PREPARATION OF IVABRADINE**
VERFAHREN ZUR HERSTELLUNG VON IVABRADIN
PROCÉDÉ POUR LA PRÉPARATION D'IVABRADINE

(30) Priority: 22.12.2008 SI 200800320
(43) Date of publication of application: 28.09.2011
(73) Proprietor: KRKA, D.D., Novo Mesto, 8501 Novo mesto (SI)
(72) Inventor: BOSE, Prosenjit, Bangalore 560 058 (IN); SIRIPALLI, Udaya Bhaskara Rao, Bangalore 560 058 (IN); KANDADAI, Appan Srinivas, Bangalore 560 058 (IN)
(74) Representative: Westendorp | Sommer
(86) International application number: PCT/EP2009/009234
(87) International publication number: WO 2010/072409

(56) References cited:
- WO-A1-2009/062377
- US-A- 5 296 482
- US-A1- 2005 261 376

## Description

### FIELD OF THE INVENTION

The present invention relates to a new process for the preparation of ivabradine comprising an optical resolution of intermediates in the preparation of ivabradine and provides diastereomeric salts.

### BACKGROUND OF THE INVENTION

Ivabradine and addition salts thereof with a pharmaceutically acceptable acid have very valuable pharmacological and therapeutic properties, especially bradycardic properties, making those compounds useful in the treatment or prevention of various clinical situations of myocardial ischemia such as angina pectoris, myocardial infarct and associated rhythm disturbances, and also in various pathologies involving rhythm disturbances, especially supraventricular rhythm disturbances, and in heart failure.

The preparation and therapeutic use of ivabradine hydrochloride have been described in EP53485 and US5296482. New crystalline forms of ivabradine hydrochloride have been described in EP1589005, EP1695710, EP1695709, EP1707562, EP1695965, EP1775288 and EP1775287. Improved processes for preparation of ivabradine are disclosed in EP1589005, EP1589014, EP1614687, EP1598333, WO200806568 and US 2005/0261376 A1.

Processes in prior art use camphor sulfonic acid or N-acetyl-L-glutamic acid for the resolution of intermediates in the preparation of ivabradine. We have found a new improved process which uses mandelic acid for resolution of an intermediate of ivabradine. In particular, the process of the present invention provides surprisingly good resolution results even on industrial scale.

### DETAILED DESCRIPTION OF THE INVENTION

The present invention relates to a process for optical resolution of a compound of formula (III) or a salt thereof 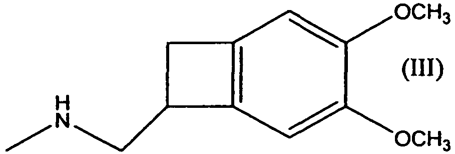 into its enantiomers by reacting compound of formula (III) with a compound of formula (A), in particular with an enantiomer of a compound of formula (A) 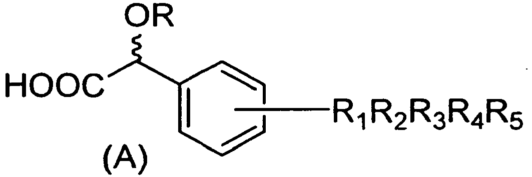 wherein R₁, R₂, R₃, R₄ and R₅ are each independently selected from the group consisting of H, alkyl, in particular straight chain alkyl having 1 to 24 carbon atoms and branched chain alkyl having 3 to 24 carbon atoms, aryl, in particular aryl having 6 to 24 carbon atoms, such as phenyl, halogen, in particular F, Cl, Br, I, OH, COOH, CN, NO₂ and SO₃H;
R is selected from the group consisting of H, alkyl, in particular straight chain alkyl having 1 to 24 carbon atoms and branched chain alkyl having 3 to 24 carbon atoms, aryl, in particular aryl having 6 to 24 carbon atoms, such as phenyl, and COR₆; R₆ is selected from the group consisting of alkyl, in particular straight chain alkyl having 1 to 24 carbon atoms and branched chain alkyl having 3 to 24 carbon atoms, and aryl, in particular aryl having 6 to 24 carbon atoms, such as phenyl.

Residues R, R₁, R₂, R₃, R₄, R₅ and R₆ can be identical or different.

More specifically, the present invention relates to a process for optical resolution of compound of formula (III) into its enantiomers that comprises the steps of:
a) treating compound (III) or a salt thereof with a compound of formula (A), in particular with an enantiomer of a compound of formula (A), in a solvent to give a diastereomeric salt of formula (IVx) 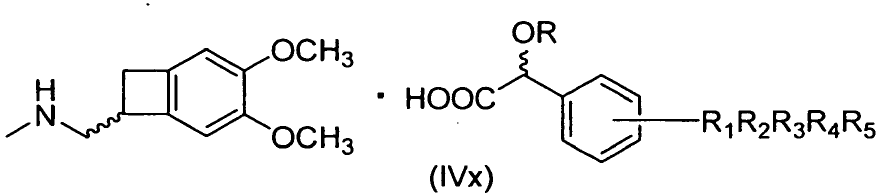 wherein R, R₁, R₂, R₃, R₄, R₅ and R₆ are as defined above.
(b) separating the diastereomeric salt of formula (IVx); and
(c) converting the diastereomeric salt of formula (IVx) to enantiomerically enriched compound of formula (III).

The term "enantiomerically enriched compound of formula (III)" encompasses e.g. mixtures of more than one stereoisomer of formula (III), in particular mixtures of two enantiomers of compound of formula (III), as well as e.g. an "enantiomerically pure compound of formula (III)", in particular (R)- enantiomer or (S)-enantiomer of compound of formula (III).

An enantiomerically enriched compound of formula (III) can be for example a mixture of enantiomers with an enantiomeric purity (enantiomeric excess; e.e.) of greater than 50%, preferably of at least 70%, and more preferably at least 90%, in particular at least 95%.

For example, in the process of the present invention, in particular in process step a) as described above, compound of formula (III) can comprise compound of formula (V), as shown below, and can be treated with an (S)-enantiomer of compound of formula (A), in particular S-(+)-mandelic acid.

In the context of the present invention, the following substituent 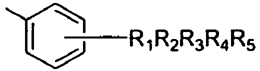 may represent

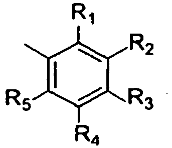

Some or all residues R₁, R₂, R₃, R₄, R₅ may be identical or different. In particular all residues R₁, R₂, R₃, R₄, R₅ may be H.

Optical resolution, in particular the step of treating compound (III) or a salt thereof with a compound of formula (A), can be performed in any suitable solvent or solvent mixture, such as the solvent selected from esters, ethers, alcohols, nitriles, amides, sulfoxides and/or any mixtures thereof. Preferably, the solvent is selected from esters, ethers and/or alcohols. More preferably, the solvent is selected from isopropyl acetate, ethyl acetate, methanol, ethanol, isopropanol, diethyl ether, and/or MTBE (Methyl Tert-Butyl Ether), as well as from solvent mixtures comprising or consisting of two or more solvents selected from isopropyl acetate, ethyl acetate, methanol, ethanol, isopropanol, diethyl ether, and MTBE. Most preferably, the solvent is selected from isopropyl acetate, ethanol, methanol MTBE and/or any mixture thereof.

To prepare the solution of a compound (III) and compound (A), the mixture is heated, preferably to reflux temperature of the solvent where it is maintained until everything is dissolved or at least compound (III) and compound (A) are both completely or partially dissolved. Optionally undissolved material may be filtered off. To start the precipitation of diastereomeric salt of formula (IV) the solution is cooled, preferably with stirring. The solution can be for example cooled to a temperature of 35°C or lower, in particular to a temperature in the range of 25 °C to -30°C, further in particular to a temperature in the range of 14°C to -10°C. Reaction mixture is filtered and the diastereomeric salt is optionally recrystallized if its optical purity is not satisfactory, what may depend for example on the further intended use, the desired product specification and requirements imposed by subsequent reaction steps. Even in absence of a recrystallization, the process of the present invention provides diastereomeric salts having a high degree of optical purity.

Optionally, the diastereomeric salt of formula (IV) can be dried after its precipitation and subsequent separation from the reaction mixture and/or its separation from the recrystallization liquid. For drying, a drying procedure or a combination of drying procedures may be chosen by a skilled person based on the general knowledge in the art and the present description.

Suitable solvents for recrystallization of diastereomeric salt can be the same as described above. In particular, recrystallization of diastereomeric salt can be carried out in any suitable solvent or solvent mixture, such as in a solvent selected from esters, ethers, alcohols, nitriles, amides, sulfoxides and/or any mixtures thereof. Preferably, the solvent is selected from esters, ethers and/or alcohols. More preferably, the solvent is selected from isopropyl acetate, ethyl acetate, methanol, ethanol, isopropanol, diethyl ether, and/or MTBE (Methyl Tert-Butyl Ether), as well as from mixtures comprising two or more solvents selected from isopropyl acetate, ethyl acetate, methanol, ethanol, isopropanol, diethyl ether, and MTBE. Most preferably, the solvent is selected from isopropyl acetate, ethanol, methanol, MTBE and/or any mixture thereof.

Optionally, diastereomeric salt can also be precipitated from the solution by the use of an antisolvent. The term "antisolvent" may pertain in particular to a fluid that, when added to a solution of a compound to be precipitated, induces a partial or complete precipitation of this compound. Preferably, an antisolvent induces the compound to be precipitated to precipitate from the solution in a greater amount or within a shorter period of time than the compound to be precipitated precipitates from a solution containing an equal concentration of the compound to be precipitated in the same solvent(s), when the solution is maintained under the same conditions, however without antisolvent addition. Antisolvents can be for example C5-C10 cyclic or acyclic saturated hydrocarbons (such as e.g. heptane and/or hexane and/or ligroin), water, and mixtures comprising C5-C10 cyclic or acyclic saturated hydrocarbons and/or water, but are not restricted to the before-mentioned antisolvents or mixtures of antisolvents.

Enantiomerically enriched compound (III) can be liberated from the diastereomeric salt by basification and extraction such as for example by treating the diastereomeric salt with basic alkaline aqueous solution and extraction with organic solvent, in particular with water insoluble organic solvent, such as for example ethyl acetate.

For example, an enantiomerically enriched compound of formula (III) may comprise at least 90 wt.%, preferably at least 98 wt.-%, more preferably at least 99 wt.%, in particular at least 99,5 wt.% of compound of formula (V), based on the total weight of enantiomerically enriched compound of formula (III).

Compound of formula (A) is preferably an enantiomer of compound of formula (A), more preferably an enantiomer of mandelic acid, such as (S)-mandelic acid or (R)-mandelic acid.

As well known to a skilled person, a respective enantiomer of a compound of formula (A) may be available under practical conditions and used for the process of the present invention for example as a mixture comprising the respective enantiomer as main component and a different enantiomer as a minor component.

The present invention relates also to a process for optical resolution of a compound of formula (III), wherein the enantiomerically enriched compound of formula (III), for example obtained in or after process step c) as described in detail above, can consist of or can comprise compound of formula (V).

Furthermore, the compound of formula (III) used as a starting material (e.g. in step a) of treating compound (III) or a salt thereof with a compound of formula (A) as described above) can comprise or consist of compound of formula (V), and/or a salt of compound of formula (III) used as a starting material (e.g. in step a) of treating compound (III) or a salt thereof with a compound of formula (A) as described above) can comprise or consist of a salt of compound of formula (V).

Moreover, the present invention encompasses a subsequent conversion of the enantiomerically enriched compound of formula (III) consisting of or comprising compound of formula (V) into intermediate compounds of the ivabradine synthesis, ivabradine, and/or a pharmaceutically acceptable salt thereof.

The process of the present invention can further comprise the step of reacting the enantiomerically enriched compound of formula (III) consisting of or comprising compound of formula (V) 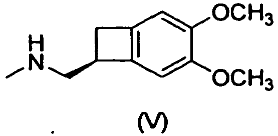 with a compound of formula (XII) 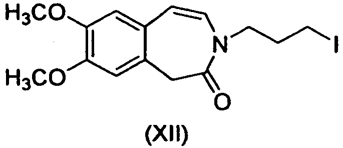 to give a compound of formula (XIII)

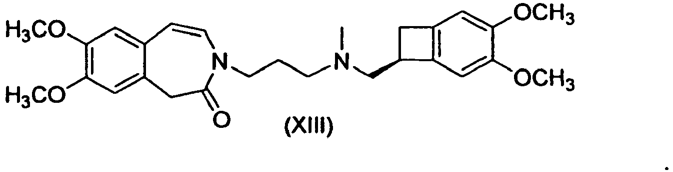

This reaction step can be carried out for example in the presence of an alkali carbonate, preferably potassium carbonate, and can be carried out for example in an organic solvent or a mixture of organic solvents, such as for example DMF (N,N-dimethylformamide).

The process of the present invention can further comprise subjecting the compound of formula (XIII) to a catalytic hydrogenation in the presence of a hydrogenation catalyst, preferably in the presence of a catalyst comprising palladium, such as palladium on carbon, to give compound of formula (XIV), as shown below.

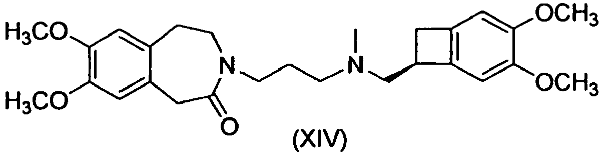

Furthermore the process of the present invention can optionally comprise reacting compound of formula (XIV) with a pharmaceutically acceptable acid to give a pharmaceutically acceptable acid addition salt of ivabradine, in particular ivabradine hydrochloride or ivabradine hydrobromide.

According to another aspect, the present invention provides compounds, in particular diastereomeric salts of formula (IV), (IVx) and (IVy), compound of formula (V), enantiomerically enriched compound of formula (III), the compound of formula (XIII), compound of formula (XIV) and pharmaceutically acceptable acid addition salts of ivabradine, which each are obtainable by processes or a sequence of process steps as described in detail before and/or in the Example section.

The present invention also relates to a diastereomeric salt of formula (IVx) 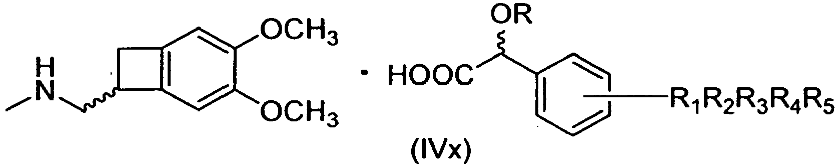 wherein R, R₁, R₂, R₃, R₄, R₅ and R₆ are as defined above.

Preferably, the diastereomeric salt of formula (IVx) is diastereomeric salt of formula (IVy)

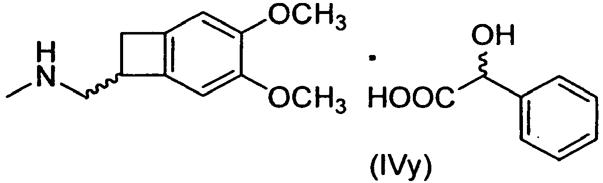

Most preferably, the diastereomeric salt of formula (IVx) is diastereomeric salt of formula (IV)

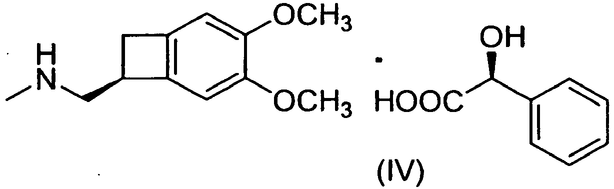

Diastereomeric salt of formula (IVx) can be in any form, amorphous or solid. Also diastereomeric salt of formula (IV) or diastereomeric salt of formula (IVy) can be in any form, amorphous or solid. Moreover, diastereomeric salt of formula (IVx), diastereomeric salt of formula (IV) and/or diastereomeric salt of formula (IVy) can be present for example in form of hydrates.

Preferably, the diastereomeric salt of formula (IVx) is in a crystalline form, such as crystalline form of diastereomeric salt of formula (IV) which is characterized by an X-ray powder diffraction pattern having peaks at about 8.3, 11.5, 16.7, 17.5,19.9 ± 0.2 degrees two-theta or by an X-ray powder diffraction pattern having peaks at about 5.3, 14.6, 15.9, 16.9, 18.1 ± 0.2 degrees two-theta.

In particular, the present invention provides the following items:
1) A process for optical resolution of a compound of formula (III) 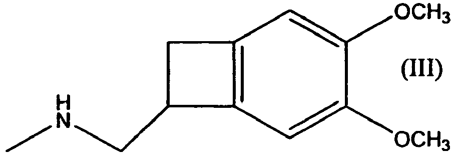 into its enantiomers which comprises reacting compound of formula (III) with an enantiomer of a compound of formula (A) 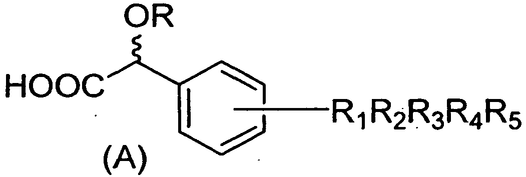 wherein R₁, R₂, R₃, R₄ and R₅ are each independently H, alkyl, aryl, halogen, OH, COOH, CN, NO₂ or SO₃H; R is H, alkyl, aryl or COR₆; R₆ is alkyl or aryl.
2) The process according to item 1, characterized in that it comprises the steps of:
   a) treating compound (III) or a salt thereof with a compound of formula (A) in a solvent to give a diastereomeric salt of formula (IVx) 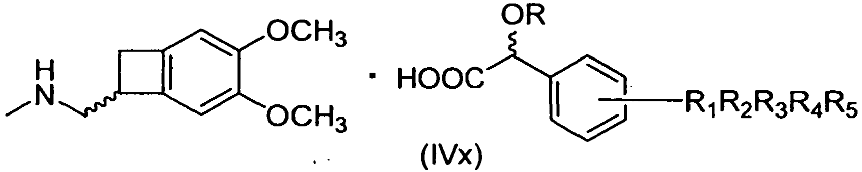 wherein R, R₁, R₂, R₃, R₄, R₅ and R₆ are as defined above.
   b) separating the diastereomeric salt of formula (IVx); and
   c) converting the diastereomeric salt of formula (IVx) to enantiomerically enriched compound of formula (III).
3) The process according to item 2, wherein the solvent is selected from esters, ethers, alcohols, nitriles, amides, sulfoxides and/or any mixtures thereof.
4) The process according to item 3, wherein the solvent is selected from isopropyl acetate, ethyl acetate, MTBE, ethanol, methanol and/or any mixtures thereof.
5) The process according to item 1, wherein the compound of formula (A) is enantiomer of mandelic acid.
6) A diastereomeric salt of formula (IVx) 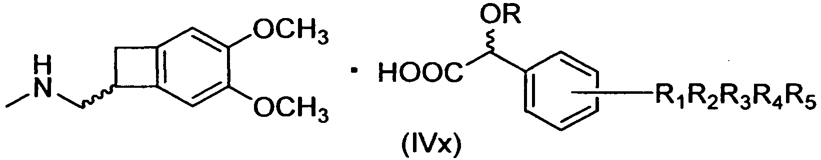 wherein R, R₁, R₂, R₃, R₄, Ra₅ and R₆ are as defined above.
7) A diastereomeric salt of formula (IVy) 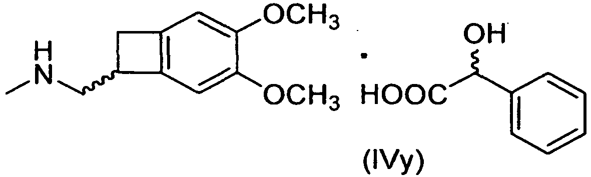
8) A diastereomeric salt of formula (IV) 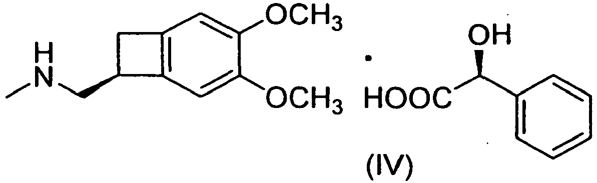
9) The diastereomeric salt according to item 6, wherein the diastereomeric salt is in an amorphous or crystalline form.
10) The diastereomeric salt according to item 8, wherein the diastereomeric salt is in a crystalline form characterized by an X-ray powder diffraction pattern having peaks at about 8.3,11.5, 16.7, 17.5, 19.9 ± 0.2 degrees two-theta.
11) The diastereomeric salt according to item 8, wherein the diastereomeric salt is in a crystalline form characterized by an X-ray powder diffraction pattern having peaks at about 5.3, 14.6, 15.9, 16.9, 18.1 ± 0.2 degrees two-theta.
12) The process according to any of items 2 to 5, wherein the compound of formula (III), in particular the compound of formula (III) subjected to a treatment with compound of formula (A) in step a) of item 2, comprises compound of formula (V), and/or
   the salt of compound of formula (III), in particular the salt of compound of formula (III) subjected to a treatment with compound of formula (A) in step a) of item 2, comprises a salt of compound of formula (V), and/or
   wherein the enantiomerically enriched compound of formula (III) comprises or consists of compound of formula (V) 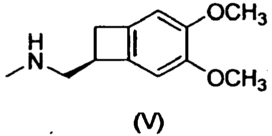
13) The process according to item 12, which process further comprises reacting the enantiomerically enriched compound of formula (III) comprising or consisting of compound of formula (V) with a compound of formula (XII) 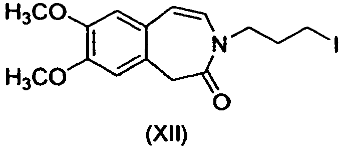 to give a compound of formula (XIII) 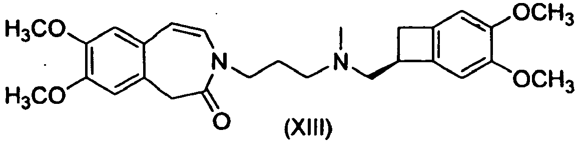
14) The process according to itemm 13, which process further comprises subjecting the compound of formula (XIII) to a catalytic hydrogenation to give a compound of formula (XIV), and 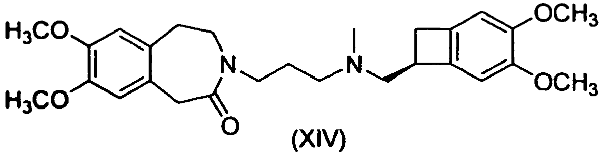
optionally reacting the compound of formula (XIV) with a pharmaceutically acceptable acid to give a pharmaceutically acceptable acid addition salt of ivabradine.

### BRIEF DESCRIPTION OF THE DRAWINGS

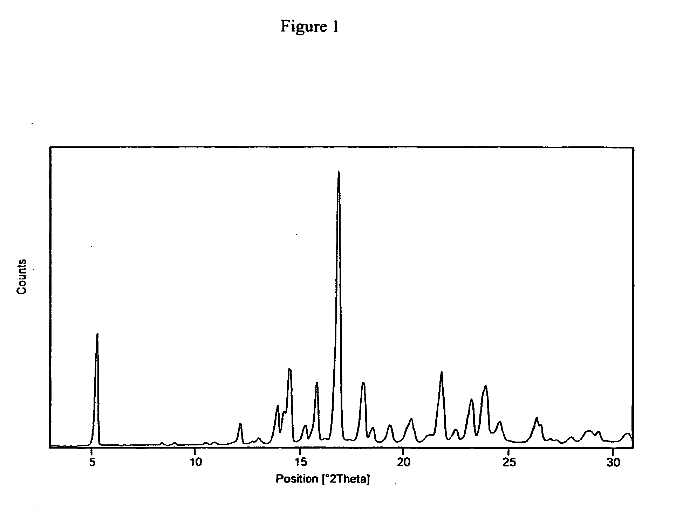

Figure 1 is an X-ray powder diffraction pattern of diastereomeric mandelate salt obtained according to example 3.

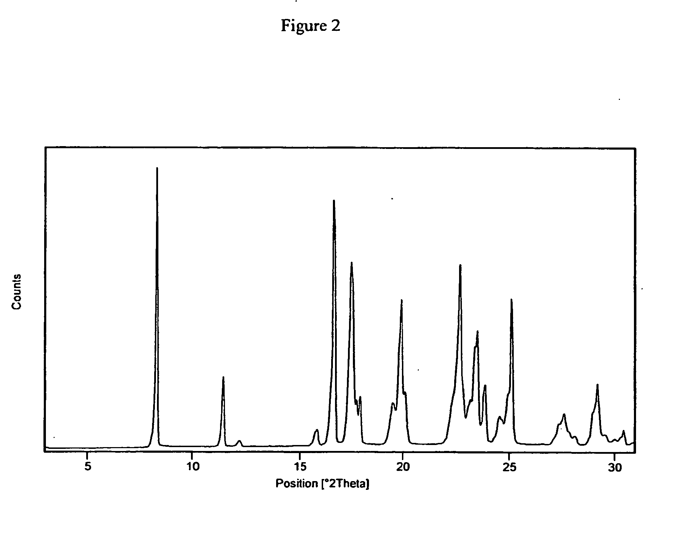

Figure 2 is an X-ray powder diffraction pattern of diastereomeric mandelate salt obtained according to example 4.

The X-ray powder diffraction pattern was obtained by Philips PW3040/60 X'Pert powder diffractometer, X'celerator detector at CuKα radiation, 1.54178 Å.

### EXAMPLES

The following examples are to further illustrate preferred aspects of the invention without limiting it thereto.

### Example 1

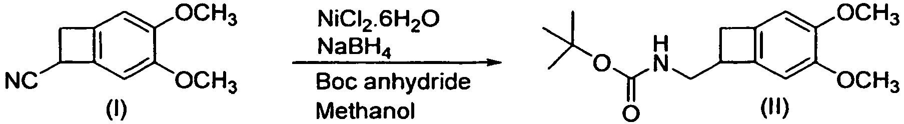

Charge 200 g of 3,4-dimethoxybicyclo [4.2.0]octa-1,3,5-triene-7-carbonitrile (I) in a 20.0 L 4-neck RB (round bottom) flask equipped with mechanical stirrer and thermometer pocket at 25°C. Add 8 L of methanol, 40.2 g of nickel chloride hexahydrate and 461.5 g of boc anhydride (Di-tert-butyl dicarbonate) at 25°C. Cool the reaction mixture at -10 to 0°C and add 280.0 g of NaBH₄ portion wise over 1 hour while maintaining the temperature of reaction mixture between -10 to 0°C. Remove the cooling bath, raise the temperature to 25°C and maintain for 2 hours. Monitor the reaction by HPLC and when the HPLC indicates the completion of the reaction (Compound (I) less than 0.2%), charge 174.6 g of diethylene triamine at 25°C. Stir for 1 hour at 25°C. Evaporate the methanol under vacuum to yield a black gummy residue. Charge 7 L of ethyl acetate in to the above residue. Charge 7 L of 10% NaHCO₃ solution. Stir for 15 minutes and separate the layers. Wash ethyl acetate layer with 7 L of water and dry the organic layer over anhydrous Na₂SO₄ and concentrate under vacuum at 50 - 55°C to obtain an off white colored solid (II).
Weight: 270. 0 g
Yield: 87.0%
HPLC purity: 96.0%

### Example 2

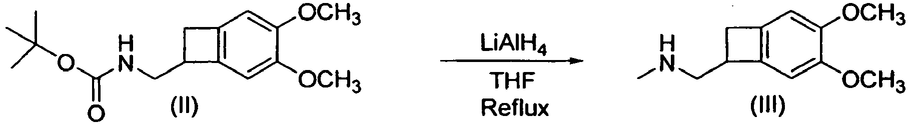

Charge 25.9 g of lithium aluminium hydride in a dry 5.0 Lt (Liter) 4-neck RB flask equipped with mechanical stirrer, condenser and thermometer pocket at 25°C. Charge 800 ml of THF slowly at 25°C and heat the above reaction mixture to reflux (75-80°C). Charge slowly the solution of 100 g of tert-butyl-3,4-dimethoxy bicyclo [4.2.0]octa-1,3,5-triene-7-carbamate (II) in 800.0 ml of THF at 78°C over a period of 1.30 hours and maintain additional 1 hour at reflux. Monitor the reaction by HPLC for completion (Compound (II) less than 0.5%) cool the reaction mixture to 25°C and further to 0°C with the help of ice cooling bath. Charge slowly 100 ml of water at 0°C. Charge slowly 20 ml of 20% sodium hydroxide solution to the above reaction mixture at 0°C to give white colored solid. Filter the solid and wash the cake with THF (2 x 500 ml). Evaporate THF under vacuum at below 50°C to obtain yellow color liquid (III).
Weight: 69.0 g
Yield: 97.7%
HPLC purity: 96.7%

### Example 3

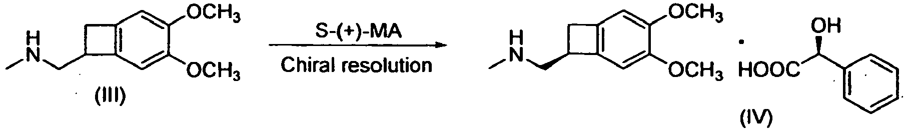

Charge 50 g of racemic [N-[[3,4-dimethoxybicyclo[4.2.0]octa-1,3,5-trien-7-yl]methyl]-N-methylamine] (III) in 3.0 L 4-neck RB flask equipped with mechanical stirrer, condenser and thermometer pocket at 25°C. Add 750 ml of isopropyl acetate and a solution of 20 g of S-(+)-mandelic acid (abbreviation: S-(+)-MA) in 221 ml of ethanol. Heat the reaction mixture to reflux i.e. 90-100°C and maintain for 30 minutes with stirring. Cool the reaction mixture with stirring to room temperature and mandelate salt (IV) will precipitate. Filter the reaction mixture to obtain crude mandelate salt (IV) and wash the solid with 50 ml of isopropyl acetate: ethanol mixture (9:1). Dry the product under vacuum at 40°C to obtain off white color solid.
Weight: 21.0. g
Yield: 56.0%
Chiral purity: S-isomer: 96.4%

### Example 4

Recrystallization of

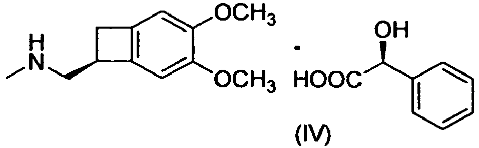

Charge 21.0 g of crude mandelate salt (IV) in a 1.0 L 4-neck RB flask equipped with mechanical stirrer and condenser and a thermometer pocket at 25°C. Add 84 ml of MTBE, 84 ml of methanol and heat to 55-60°C and maintain for 30 minutes. Charge 63 ml of MTBE at 55-60°C, wait 5 minutes and cool the reaction mixture to 25°C and maintain at the same temperature for overnight. Filter the precipitate and wash with MTBE (2 x 25 ml). Dry the product under vacuum at 40°C to obtain white color solid (IV).
Weight: 17.5 g
Chiral purity: S-isomer: 99.7%

For second crop transfer the filtrate to a RB flask using methanol and evaporate to dryness to get 3.5 g of crude mandelate salt (IV). Recrystallize as described above.
Weight: 1.5 g
Chiral purity: S-isomer: 99.81%
Total Weight: 17.5 g + 1.5 g = 19.0 g

### Example 5

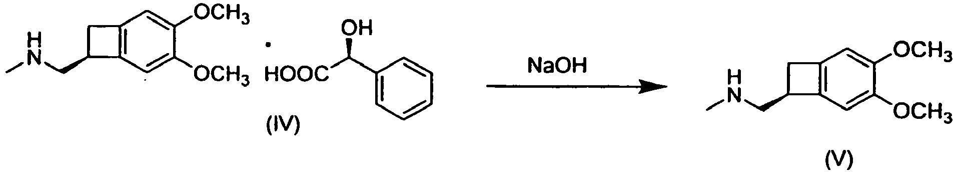

Stir 19.0 g of mandelate salt (IV), 190 ml of ethyl acetate and 190 ml of 2N aqueous NaOH solution for 10 minutes at 25°C. Separate the organic and aqueous layers and wash the organic layer with water. Dry the organic layer over anhydrous Na₂SO₄ and evaporate ethyl acetate under vacuum at below 50°C to get a yellow liquid (V).
Yield: 10.5 g
HPLC purity: 99.4%
Chiral purity: S-isomer: 99.8%

### Example 6

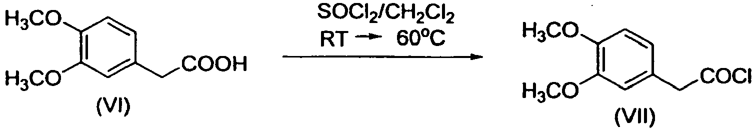

Charge 100.0 g of 3,4-Dimethoxy phenyl acetic acid (VI) and 110 ml of dichloromethane in a 500.0 ml 4-neck RB flask equipped with mechanical stirrer, condenser and thermometer pocket at 25°C. Slowly add 177.7 g of thionyl chloride at 25°C over period of 15 minutes and maintain for additional 15 minutes at 25°C. Heat it to 45-50°C and maintain for 15 minutes. Monitor the reaction by TLC for completion (Compound VI less than 1.0%, mobile phase - ethylacetate: hexane = 50:50). Distill off dichloromethane and excess thionyl chloride using normal distillation (vacuum at the end of the distillation for 5 min to remove the traces of thionyl chloride) to obtain a residue.
Weight: 130.0 g

### Example 7

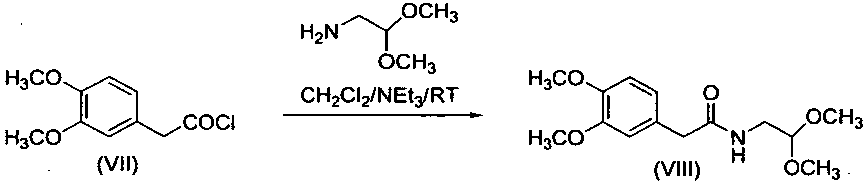

Charge 53.4 g of aminoacetaldehyde dimethyl acetal, 427 ml of dichloromethane and 77.1 g of triethylamine in a 2.0 L 4-neck RB flask equipped with mechanical stirrer, dropping funnel and thermometer pocket at 25°C. Cool the reaction mixture to 10°C with water ice-bath cooling. Add to above reaction mixture 130.0 g of 3,4-dimethoxy phenyl acetoxy chloride (VII) in 218 ml dichloromethane drop wise for 30 minutes at 10°C. Stir for 1 hour at 25°C. Monitor the reaction by HPLC (Compound VII should be less than 3.0%). After completion add 800 ml of water and stir for 10 minutes. Separate the layers and wash the dichloromethane layer with 500 ml of 10% sodium bicarbonate solution and with 500 ml of water. Dry the organic layer over anhydrous Na₂SO₄ and evaporate dichloromethane under vacuum at below 40°C to give a brown colored residue (VIII).
Weight: 142.0 g

### Example 8

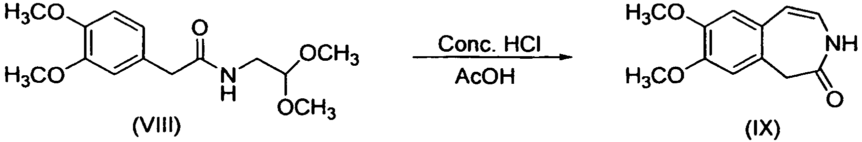

Charge 142.0 g of N-(2,2-dimethoxyethyl)- 2-(3,4-dimethoxyphenyl) acetamide (VIII), 710 ml of glacial acetic acid and 710 ml of conc. hydrochloric acid in a 3.0 L RB flask equipped with mechanical stirrer and thermometer pocket at 25°C. Stir for 17 hours at 25°C. Monitor the reaction by HPLC (Compound VIII should be less than 4.0%). If HPLC complies, pour the reaction mixture on 3.0 kg of crushed ice. Stir for 30 minutes and filter the solid material. Wash with 1 L of water, suck dry and unload the material. Dry the material (IX) under vacuum at 55-60°C till the LOD comes to below 0.5%.
Weight: 51.0 g
Yield: 77.5%
HPLC purity: 98.9%

### Example 9

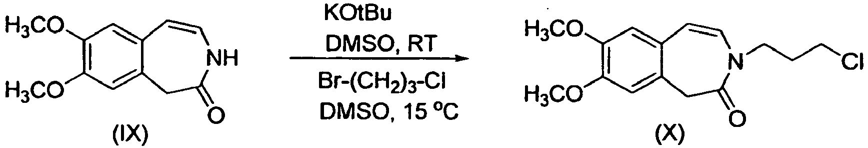

a) Charge 50.0 g of 7,8-dimethoxy-1,3-dihydro-2H-3-benazepin-2-one (IX), 350.0 ml of DMSO and 30.7 g of potassium tert-butoxide in a 4-neck RB flask equipped with mechanical stirrer, thermometer pocket and septum at 25°C. Stir for 30 minutes at 25°C.
b) Charge 43.1 g of 1-bromo-3-chloro propane and 108.0 ml of DMSO in a separate RB flask equipped with septum at 25°C. Cool the reaction mixture to 15-18°C with water ice-bath cooling.

Charge the reaction mixture a) to reaction mixture b) through cannula over a period of 20 minutes at 15-18°C.

Stir for another 30 minutes at 15-18°C. Monitor the reaction by HPLC (Compound IX should be less than 1.0%). If HPLC complies, pour the reaction mixture on 3.0 kg of crushed ice. Stir for 3-4 hour till the product crystallizes completely. Filter the pale brown colored precipitate. Wash with water (2 x 500 ml). Dry the material (X) under vacuum at 45-50 °C till the LOD comes to below 0.5%.
Weight: 57.1 g
Yield: 84.6 %
HPLC purity: 94.0%

### Example 10

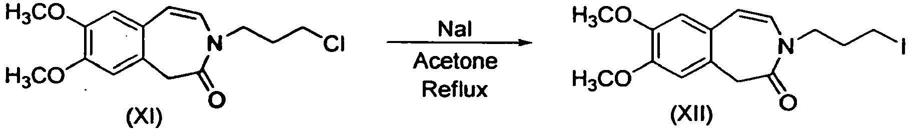

Charge 55.0 g of [3-(3-chloropropyl)-7,8-dimethoxy-1,3-dihydro-2H-3-benzazepine-2-one] (XI), 330 ml of acetone and 55.74 g of sodium iodide in a RB flask equipped with mechanical stirrer, condenser and thermometer pocket at 25°C. Heat the reaction mixture to 60-65°C. Reflux for 30 hours with stirring and monitor the reaction by HPLC. If starting compound (XI) remains additional NaI is added and continue refluxing till compound (XI) comes below 1.0%. Evaporate the acetone and add 225 ml of dichloromethane at 25°C and stir for 10 minutes. Filter the solid and wash the solid with dichloromethane (2 x 100 ml). Combine the dichloromethane solutions and charge 400 ml of 5% sodium thiosulfate solution and stir for 10 minutes. Separate the layers and wash the organic layer with 400 ml of water. Dry the organic layer over anhydrous Na₂SO₄ and evaporate dichloromethane under vacuum at below 40°C to result a brown colored residue. Weight: 55.0 g

Charge 110 ml of acetone to the above brown colored residue and stir for 30 minutes. Filter the precipitated material and wash with 25 ml of acetone and dry under vacuum to give off white colored solid. Charge 100 ml of acetonitrile to the above obtained off white solid. Stir for 30 minutes and filter the precipitate. Wash with 25 ml acetonitrile to give off-white colored solid (XII). Dry the compound at 50°C under vacuum for 12 hours.
Weight: 37. 0 g
Yield: 51. 4 %
HPLC purity: 98. 3%

### Example 11

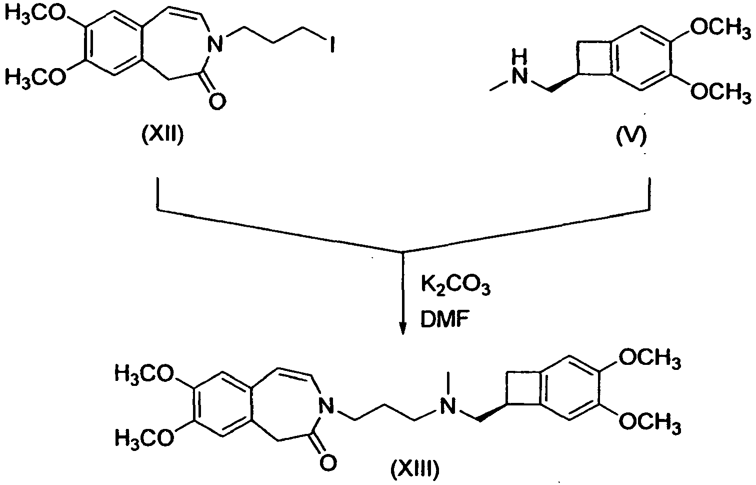

Charge 21.0 g of [N-[[(7S)-3,4-dimethoxybicyclo[4.2.0]octa-1,3,5-trien-7-yl]methyl]-N-methylamine (V), 43.15 g of 3-(3-iodopropyl)-7,8-dimethoxy-1,3-dihydro-2H-3-benzazepine-2-one (XII), 60.0 g of K₂CO₃ and 315 ml of DMF in a RB flask equipped with mechanical stirrer and thermometer pocket at 25°C. Heat the reaction mixture to 40-45°C and maintain at the same temperature for 12 hours. Monitor the reaction by HPLC. (if starting material i.e. compound (V) remains, add compound (XII) and continue heating with stirring till compound (V) comes to less than 1.0%). If HPLC complies, cool the reaction mixture to 25°C. Charge 1500 ml of 1N HCl (pH should be acidic), 1500 ml of ethyl acetate (3 x 500 ml) and stir for 10 minutes and separate the layers. Charge 10% NaOH solution to aqueous layer till pH of the solution becomes basic (pH ~ 7.0 - 8.0). Charge ethyacetate (2 x 500.0 ml), stir for 10 minutes and separate the layers. Wash the ethyl acetate layer with 500 ml of water. Dry ethyl acetate layer over anhydrous Na₂SO₄ and evaporate ethyl acetate under vacuum at below 50°C to result a brown colored residue (XIII).
Weight: 43.0 g
Yield: 91.0 %
HPLC purity (Reverse phase): >98.5%

### Example 12

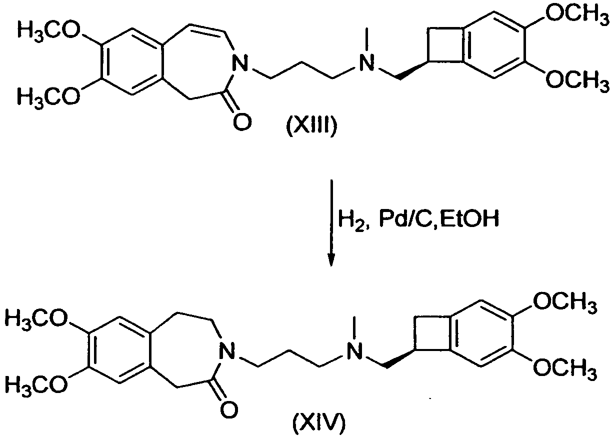

a) Charge 10.0 g of 3-[3-[[[(7S)-3,4-Dimethoxybicyclo[4.2.0]octa-1,3,5-trien-7-yl]methyl]methylamino]propyl]-1,3-dihydro-7,8-dimethoxy-2H-3-benzazepin-2-one (XIII) and 300 ml of ethanol in a hydrogenation vessel (Parr hydrogenation apparatus) at 25°C. Cool the above solution to 105°C Charge 5.0 g of 10% palladium on carbon at 105°C. Pass hydrogen gas (3,45 bar (50 psi) into the reaction mixture at 60°C for 2 hours. Monitor the reaction by HPLC (compound (XIII) should be less than 0.5%). If HPLC complies, filter the Pd/C using celite. Wash the celite bed with ethanol (2 x 50.0 ml). Evaporate the ethanol completely under vacuum at below 50°C to get a gummy solid (XIV). (Weight = 9.0 g)

### Example 13: Ivabradine hydrochloride

Charge 45.0 ml of methanol to the 9.0 g of the above solid (XIV) at 25°C. Charge 10.0 ml of methanolic HCl (10% w/w) at 10-15°C. Stir for 1 hour at 10-15°C. Evaporate the methanol under vacuum at below 50°C to dryness. Charge 36.0 ml of acetonitrile at 25°C and stir for 30 minutes. Filter the solid at 25°C under nitrogen through sintered flask. Wash with acetonitrile (2 x 10 ml). Dry the material under vacuum at 55-60°C till the LOD comes to below 0.5%.
Weight: 6.5 g of ivabradine hydrochloride
Yield: 60.1 %
HPLC purity (Reverse phase): >99.3%
Chiral purity: 99. 8%

### Example 14: Ivabradine hydrobromide

a) 14.5 ml (0.128 mole) 48 % aqueous solution of hydrogen bromide is added to the solution of 60.0 g (0.128 mole) ivabradine in 600 ml of methylene chloride at 20-25°C. The solvent is evaporated under vacuum to dryness. 400 ml of ethyl acetate is added to residue and maintained for 12 hours at 20-25°C. The product is filtered, washed with 50 ml of ethyl acetate and dried in a vacuum dryer at final temperature 50°C. During drying the product is sieved to deagglomerate lumps. 61.5 g of crystalline ivabradine hydrobromide hydrate is obtained (assay of water 3.6 %).
b) 1.15 ml (0.01 mole) 48 % aqueous solution of hydrogen bromide is added to the solution of 4.7 g (0.01 mole) ivabradine in 50 ml of ethyl acetate at 20-25°C. The suspension is heated to reflux (76°C), stirred for 10 minutes and gradually cooled to 20-25°C. 400 ml of ethyl acetate is added to residue and maintained for 12 hours at 20-25°C. The product is filtered, washed with 4 ml of ethyl acetate and dried in a vacuum dryer at final temperature 50°C. 3.2 g of crystalline ivabradine hydrobromide hydrate is obtained (assay of water 3.7 %).
c) 2.3 ml (0.02 mole) 48 % aqueous solution of hydrogen bromide is added to the solution of 9.4 g (0.02 mole) ivabradine in 100 ml of methylene chloride at 20-25°C. The solvent is evaporated under vacuum to dryness to obtain 10.9 g of amorphous ivabradine hydrobromide (assay of water 8.4 %).

## Claims

1. A process for optical resolution of a compound of formula (III) or a salt thereof 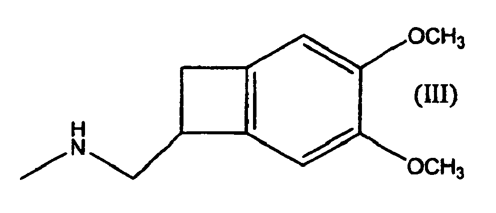 into its enantiomers which comprises reacting compound of formula (III) with an enantiomer of a compound of formula (A) 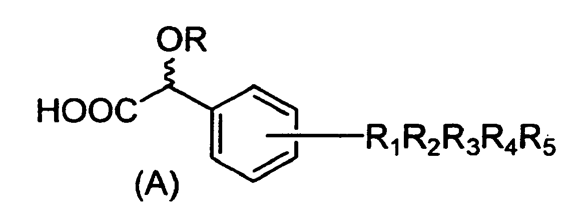 wherein R₁, R₂, R₃, R₄ and R₅ are each independently selected from the group consisting of H, alkyl, aryl, halogen, OH, COOH, CN, NO₂ and SO₃H; R is selected from the group consisting of H, alkyl, aryl and COR₆; R₆ is alkyl or aryl.

2. The process according to claim 1, **characterized in that** it comprises the steps of:
a) treating compound (III) or a salt thereof with a compound of formula (A) in a solvent to give a diastereomeric salt of formula (IVx) 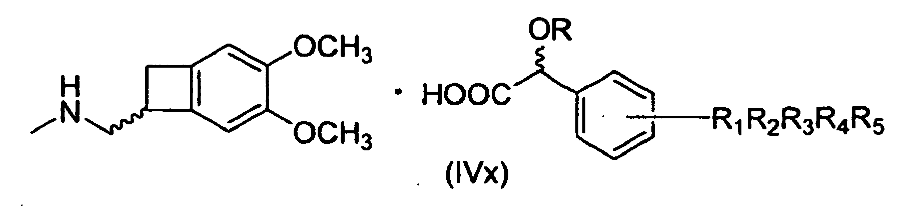 wherein R, R₁, R₂, R₃, R₄, R₅ and R₆ are as defined above in claim 1.
b) separating the diastereomeric salt of formula (IVx); and
c) converting the diastereomeric salt of formula (IVx) to enantiomerically enriched compound of formula (III).

3. The process according to claim 2, wherein the solvent is selected from esters, ethers, alcohols, nitriles, amides, sulfoxides and/or any mixtures thereof.

4. The process according to claim 3, wherein the solvent is selected from isopropyl acetate, ethyl acetate, MTBE, ethanol, methanol and/or any mixtures thereof.

5. The process according to any of the preceding claims, wherein compound of formula (A) is enantiomer of mandelic acid.

6. A diastereomeric salt of formula (IVx) 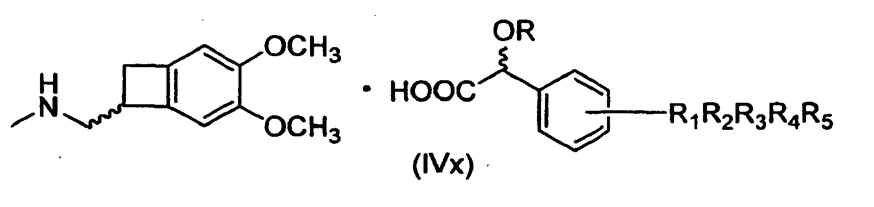 wherein R, R₁, R₂, R₃, R₄, R₅ and R₆ are as defined above in claim 1.

7. The diastereomeric salt of claim 6, which is a diastereomeric salt of formula (IVy)

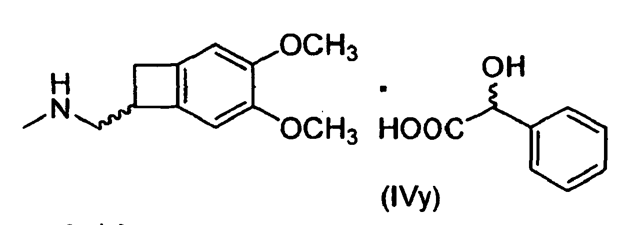

8. A diastereomeric salt of claims 6 or 7 of formula (IV)

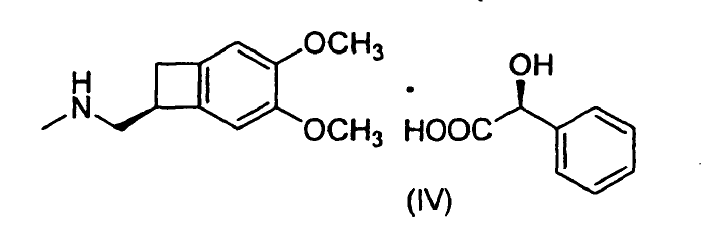

9. The diastereomeric salt according to any of claims 6 to 8, wherein the diastereomeric salt is in an amorphous or crystalline form.

10. The diastereomeric salt according to claim 8, wherein the diastereomeric salt is in a crystalline form **characterized by** an X-ray powder diffraction pattern having peaks at 8.3, 11.5, 16.7, 17.5, 19.9 ± 0.2 degrees two-theta.

11. The diastereomeric salt according to claim 8, wherein the diastereomeric salt is in a crystalline form **characterized by** an X-ray powder diffraction pattern having peaks at 5.3, 14.6, 15.9, 16.9, 18.1 ± 0.2 degrees two-theta.

12. The process according to any of claims 2 to 5, wherein the compound of formula (III), in particular the compound of formula (III) subjected to a treatment with compound of formula (A) in step a) of claim 2, comprises compound of formula (V), and/or
the salt of compound of formula (III), in particular the salt of compound of formula (III) subjected to a treatment with compound of formula (A) in step a) of claim 2, comprises a salt of compound of formula (V), and/or
wherein the enantiomerically enriched compound of formula (III) comprises or consists of compound of formula (V)

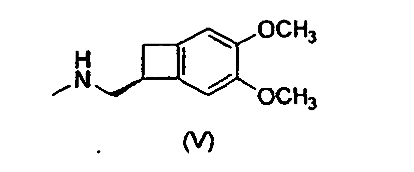

13. The process according to claim 12, which process further comprises reacting the enantiomerically enriched compound of formula (III) comprising or consisting of compound of formula (V) with a compound of formula (XII) 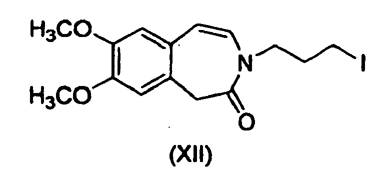 to give a compound of formula (XIII)

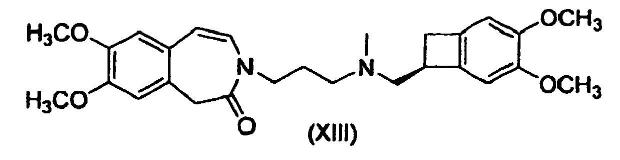

14. The process according to claim 13, which process further comprises subjecting the compound of formula (XIII) to a catalytic hydrogenation to give a compound of formula (XIV), and 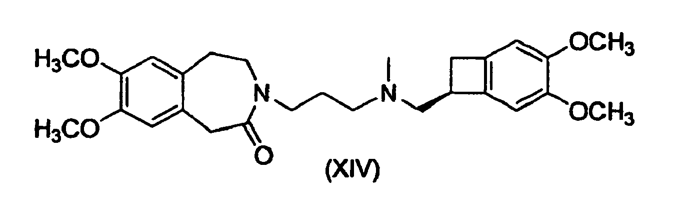 optionally reacting the compound of formula (XIV) with a pharmaceutically acceptable acid to give a pharmaceutically acceptable acid addition salt of ivabradine.

## Patentansprüche

1. Verfahren zur optischen Auftrennung einer Verbindung der Formel (III) oder eines Salzes davon 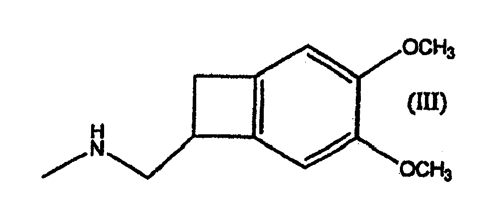 in ihre Enantiomere, welches Umsetzen einer Verbindung der Formel (III) mit einem Enantiomer einer Verbindung der Formel (A) umfasst 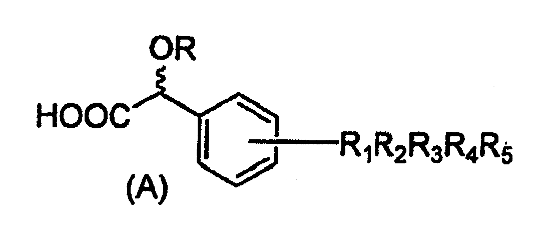 wobei R₁, R₂, R₃, R₄ und R₅ jeweils unabhängig aus der Gruppe, bestehend aus H, Alkyl, Aryl, Halogen, OH, COOH, CN, NO₂ und SO₃H, ausgewählt sind; R aus der Gruppe, bestehend aus H, Alkyl, Aryl und COR₆, ausgewählt ist; R₆ Alkyl oder Aryl ist.

2. Verfahren gemäß Anspruch 1, **dadurch gekennzeichnet, dass** es die folgenden Schritte umfasst:
a) Behandeln einer Verbindung (III) oder eines Salzes davon mit einer Verbindung der Formel (A) in einem Lösungsmittel, wobei ein diastereomeres Salz der Formel (IVx) erhalten wird 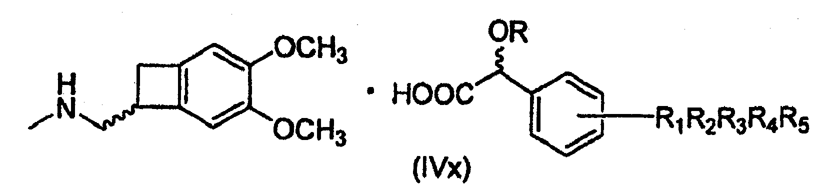 wobei R, R₁, R₂, R₃, R₄, R₅ und R₆ wie vorstehend in Anspruch 1 definiert sind,
b) Auftrennen des diastereomeren Salzes der Formel (IVx); und
c) Umwandeln des diastereomeren Salzes der Formel (IVx) in eine enantiomer angereicherte Verbindung der Formel (III).

3. Verfahren gemäß Anspruch 2, wobei das Lösungsmittel aus Estern, Ethern, Alkoholen, Nitrilen, Amiden, Sulfoxiden und/oder jedweden Gemischen davon ausgewählt ist.

4. Verfahren gemäß Anspruch 3, wobei das Lösungsmittel aus Isopropylacetat, Ethylacetat, MTBE, Ethanol, Methanol und/oder jedweden Gemischen davon ausgewählt ist.

5. Verfahren gemäß einem der vorhergehenden Ansprüche, wobei eine Verbindung der Formel (A) ein Enantiomer von Mandelsäure ist.

6. Diastereomeres Salz der Formel (IVx) 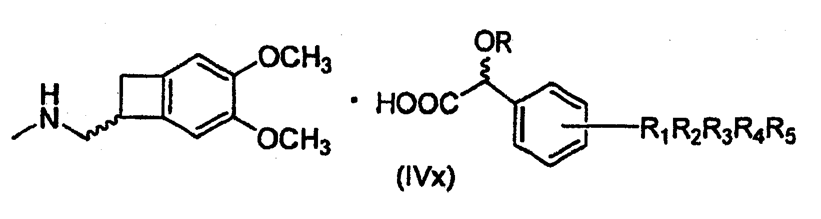 wobei R, R₁, R₂, R₃, R₄, R₅ und R₆ wie vorstehend in Anspruch 1 definiert sind.

7. Diastereomeres Salz nach Anspruch 6, welches ein diastereomeres Salz der Formel (IVy) ist

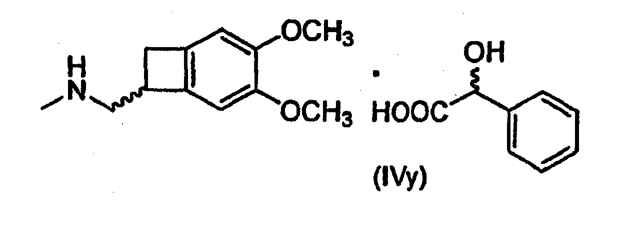

8. Diastereomeres Salz nach den Ansprüchen 6 oder 7 der Formel (IV)

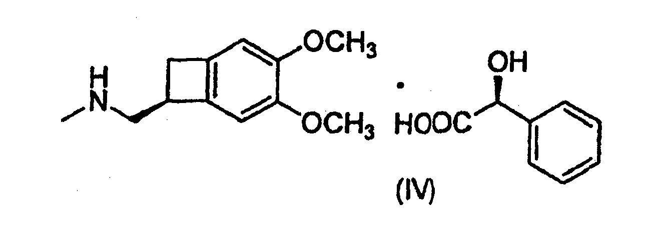

9. Diastereomeres Salz gemäß einem der Ansprüche 6 bis 8, wobei das diastereomere Salz in einer amorphen oder kristallinen Form vorliegt.

10. Diastereomeres Salz gemäß Anspruch 8, wobei das diastereomere Salz in einer kristallinen Form, charakterisiert durch ein Röntgenpulverbeugungsmuster mit Peaks bei 8,3, 11,5, 16,7, 17,5, 19,9 ± 0,2 Grad zwei-Theta, vorliegt.

11. Diastereomeres Salz gemäß Anspruch 8, wobei das diastereomere Salz in einer kristallinen Form, charakterisiert durch ein Röntgenpulverbeugungsmuster mit Peaks bei 5,3, 14,6, 15,9, 16,9,18,1 ± 0,2 Grad zwei-Theta, vorliegt.

12. Verfahren gemäß einem der Ansprüche 2 bis 5, wobei die Verbindung der Formel (III), insbesondere die Verbindung der Formel (III), welche einer Behandlung mit einer Verbindung der Formel (A) in Schritt a) von Anspruch 2 ausgesetzt wird, eine Verbindung der Formel (V) umfasst, und/oder
das Salz einer Verbindung der Formel (III), insbesondere das Salz einer Verbindung der Formel (III), welches einer Behandlung mit einer Verbindung der Formel (A) in Schritt a) von Anspruch 2 ausgesetzt wird, ein Salz einer Verbindung der Formel (V) umfasst, und/oder
wobei die enantiomer angereicherte Verbindung der Formel (III) eine Verbindung der Formel (V) umfasst oder daraus besteht

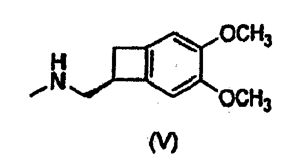

13. Verfahren gemäß Anspruch 12, wobei das Verfahren ferner umfasst Umsetzen der enantiomer angereicherten Verbindung der Formel (III), umfassend oder bestehend aus einer Verbindung der Formel (V), mit einer Verbindung der Formel (XII) 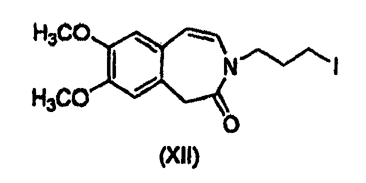 wobei eine Verbindung der Formel (XIII) erhalten wird

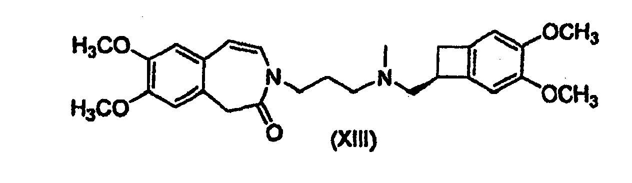

14. Verfahren gemäß Anspruch 13, wobei das Verfahren ferner umfasst Aussetzen der Verbindung der Formel (XIII) einer katalytischen Hydrierung, wobei eine Verbindung der Formel (XIV) erhalten wird, und 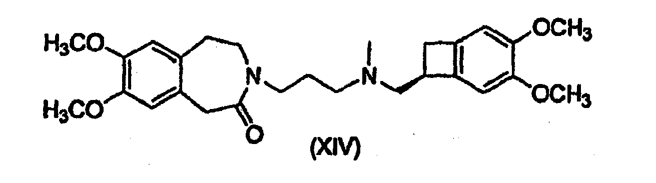 gegebenenfalls Umsetzen der Verbindung der Formel (XIV) mit einer pharmazeutisch verträglichen Säure, wobei ein pharmazeutisch verträgliches Säureadditionssalz von Ivabradin erhalten wird.

## Revendications

1. Procédé de résolution optique d'un composé de formule (III) ou d'un sel de celui-ci : 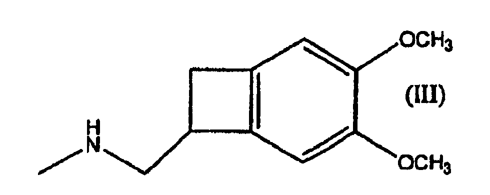 en ses énantiomères, qui comprend la réaction du composé de formule (III) avec un énantiomère d'un composé de formule (A) : 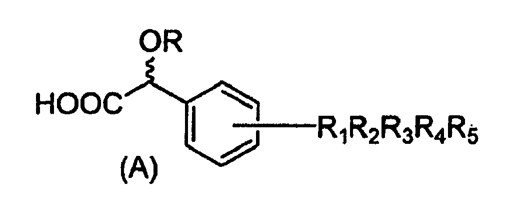 où R₁, R₂, R₃ , R₄ et R₅ sont chacun indépendamment choisis parmi le groupe consistant en H, alkyle, aryle, halogène, OH, COOH, CN, NO₂ et SO₃H ; R est choisi parmi le groupe consistant en H, alkyle, aryle et COOR₆, R₆ est alkyle ou aryle.

2. Procédé selon la revendication 1, **caractérisé en ce qu'**il comprend les étapes de :
a) traitement du composé (III) ou d'un sel de celui-ci avec un composé de formule (A) dans un solvant pour donner un sel diastéréoisomérique de formule (IVx) : 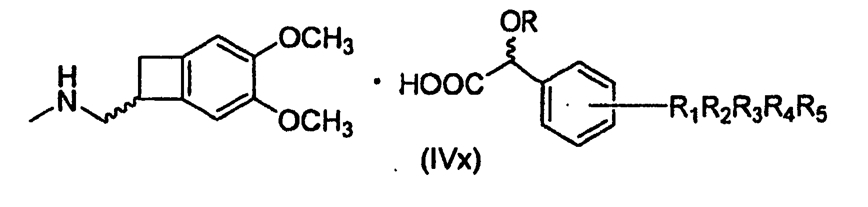 où R, R₁, R₂, R₃, R₄, R₅ et R₆ sont tels que définis ci-dessus à la revendication 1 ;
b) séparation du sel diastéréoisomérique de formule (IVx), et
c) conversion du sel diastéréoisomérique de formule (IVx) en un composé énantiomériquement enrichi de formule (III).

3. Procédé selon la revendication 2, où le solvant est choisi parmi des esters, des éthers, des alcools, des nitriles, des amides, des sulfoxydes et/ou tout mélange de ceux-ci.

4. Procédé selon la revendication 3, où le solvant est choisi parmi l'acétate d'isopropyle, l'acétate d'éthyle, le MTBE, l'éthanol, le méthanol et/ou tout mélange de ceux-ci.

5. Procédé selon l'une quelconque des revendications précédentes, où le composé de formule (A) est un énantiomère de l'acide mandélique.

6. Sel diastéréoisomérique de formule (IVx) : 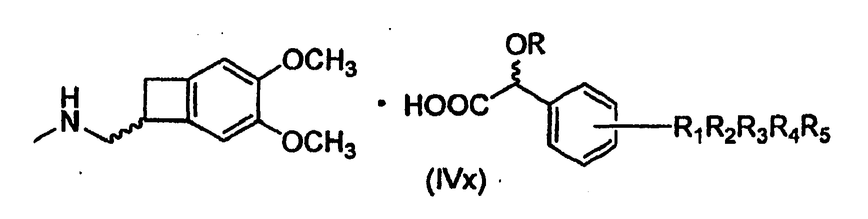 où R, R₁, R₂, R₃, R₄, R₅ et R₆ sont tels que définis ci-dessus à la revendication 1.

7. Sel diastéréoisomérique selon la revendication 6, qui est un sel diastéréoisomérique de formule (IVy) :

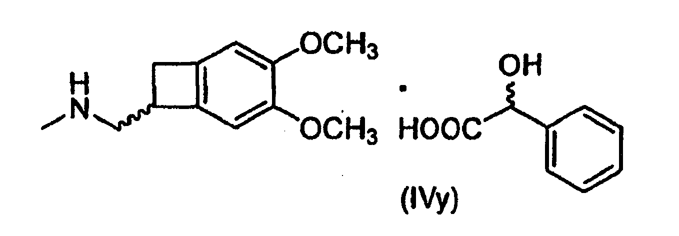

8. Sel diastéréoisomérique selon la revendication 6 ou 7, de formule (IV) :

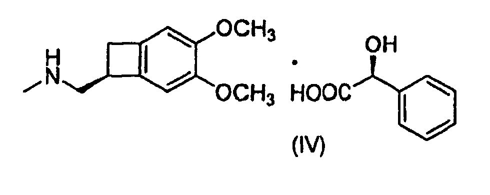

9. Sel diastéréoisomérique selon l'une quelconque des revendications 6 à 8, où le sel diastéréoisomérique est sous forme amorphe ou cristalline.

10. Sel diastéréoisomérique selon la revendication 8, où le sel diastéréoisomérique est sous une forme cristalline, **caractérisée par** un schéma de diffraction des rayons X de poudre, ayant des pics en 8,3, 11,5, 16,7, 17,5, 19,9 ± 0,2 degrés deux-thêta.

11. Sel diastéréoisomérique selon la revendication 8, où le sel diastéréoisomérique est sous une forme cristalline, **caractérisée par** un schéma de diffraction des rayons X de poudre, ayant des pics en 5,3, 14,6, 15,9, 16,9, 18,1 ± 0,2 degrés deux-thêta.

12. Procédé selon l'une quelconque des revendications 2 à 5, où le composé de formule (III), en particulier le composé de formule (III) soumis à un traitement avec le composé de formule (A) à l'étape a) de la revendication 2, comprend un composé de formule (V) et/ou le sel du composé de formule (III), en particulier le sel du composé de formule (III) soumis à un traitement avec le composé de formule (A) à l'étape a) de la revendication 2, comprend un sel du composé de formule (V), et/ou
où le composé de formule (III) énantiomériquement enrichi comprend ou consiste en un composé de formule (V) :

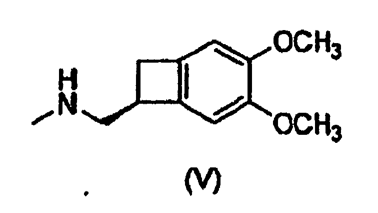

13. Procédé selon la revendication 12, où le procédé comprend en outre, la réaction du composé de formule (III) énantiomériquement enrichi comprenant ou consistant en le composé de formule (V), avec un composé de formule (XII) : 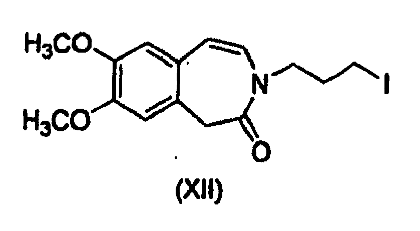 pour donner un composé de formule (XIII) :

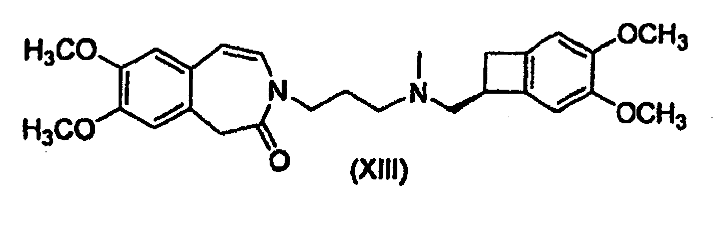

14. Procédé selon la revendication 13, où le procédé comprend en outre, le fait de soumettre le composé de formule (XIII) à une hydrogénation catalytique pour donner un composé de formule (XIV), et 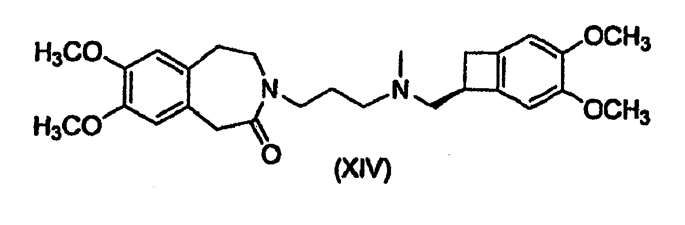 et le cas échéant, la réaction du composé de formule (XIV) avec un acide pharmaceutiquement acceptable pour donner un sel d'addition d'acide pharmaceutiquement acceptable d'ivabradine.
